# EUROPÄISCHE PATENTSCHRIFT

(11) **EP 0 554 642 B1**
(45) Veröffentlichungstag und Bekanntmachung des Hinweises auf die Patenterteilung: **10.07.1996**
(21) Anmeldenummer: 92810937.0
(22) Anmeldetag: 01.12.1992
(51) Int. Cl.: D02G 1/12, G01N 33/36

(54) **Verfahren und Vorrichtung zum kontinuierlichen Kräuseln von thermoplastischen Fäden**
Method and apparatus for continual crimping of thermoplastic yarns
Procédé et appareil pour le frisage en continu de fils thermoplastique

(30) Priorität: 07.02.1992 CH 359/92; 30.06.1992 CH 2052/92
(43) Veröffentlichungstag der Anmeldung: 11.08.1993
(73) Patentinhaber: MASCHINENFABRIK RIETER AG, CH-8406 Winterthur (CH)
(72) Erfinder: Nabulon, Werner, CH-8455 Rüdlingen (CH); Maier, Jörg, CH-8500 Frauenfeld (CH); Grossenbacher, Peter, CH-8400 Winterthur (CH); Graf, Felix, CH-8400 Winterthur (CH); Wirz, Armin, CH-8475 Ossingen (CH)
(74) Vertreter: Frei, Alexandra Sarah

(56) Entgegenhaltungen:
- EP-A- 0 212 175
- EP-A- 0 428 045
- DD-A- 0 214 159
- US-A- 4 067 092
- US-A- 4 188 691

## Beschreibung

Die Erfindung liegt auf dem Gebiete der Textiltechnik und betrifft ein Verfahren und eine Vorrichtung zum kontinuierlichen Kräuseln von Fäden aus einem thermoplastischen Material gemäss den Oberbegriffen der entsprechenden unabhängigen Patentansprüche bekannt beispielweise aus dem Dokument EP-A-0 212 175.

Zum kontinuierlichen Kräuseln von Fäden (Fibrillenbündel) aus thermoplastischem Material werden diese beispielsweise mit Hilfe eines unter Druck strömenden, heissen Fördermediums durch einen Förderkanal bewegt, dabei erhitzt und dann durch eine Düsenaustrittsöffnung in eine Stauchkammer gefördert, wobei die Stauchkammer derart gestaltet ist, dass sich das Fördermedium beim Austritt aus der Düsenaustrittsöffnung entspannt. Der Faden prallt in der Stauchkammer auf einen durch bereits aus der Öffnung ausgetretenen Faden gebildeten Pfropfen, wobei er gekräuselt wird. Der Pfropfen wird weitergefördert mit einer Geschwindigkeit, die kleiner ist als die Fadengeschwindigkeit im Förderkanal, wird dann abgekühlt und weiter zu einem texturierten Garn aufgelöst.

Die Stauchkammer, in der sich der Pfropfen bildet, kann begrenzt sein durch stationäre, den Pfropfen längsseitig umgebende, durchbrochene Wandungen, die beispielsweise aus Lamellen bestehen. Der Pfropfen wird durch den Staudruck des Fördermittels gegen die Reibung an den Stauchkammerwandungen durch die Stauchkammer gestossen und verlässt diese durch eine der Düsenaustrittsöffnung gelgenüberliegende Pfropfenöffnung, die mit einem den Pfropfen dosiert austragenden Förderwalzenpaar versehen sein kann.

Die Stauchkammer kann auch nur teilweise von stationären Wandungen begrenzt sein und teilweise von Wandungen, die sich mit Pfropfengeschwindigkeit bewegen. Ein Verfahren und eine Vorrichtung zum kontinuierlichen Kräuseln von thermoplastischen Fäden mit einer Stauchkammer, die teilweise mit dem Pfropfen bewegende Wände aufweist, ist beispielsweise beschrieben in der europäischen Patentschrift Nr. 310890 derselben Anmelderin. Die beschriebene Vorrichtung weist eine Texturierdüse auf mit einem Förderkanal, einer Düsenaustrittsöffnung und zwei sich von dieser in Fadenlaufrichtung erstreckenden Anformungen, die die stationären Wandungsteile der Stauchkammer bilden. Zum Weitertransport des zwischen den Anformungen gebildeten Pfropfens dient ein durch seitliche Führungsmittel gebildeter, um den Umfang einer rotierenden Pfropfenförderwalze verlaufender Kanal, in den die Anformungen reichen. Die seitlichen Führungsmittel dieses Kanales stellen im Bereiche der Düsenaustrittsöffnung die mit dem Pfropfen bewegenden Teile der Stauchkammerwandungen dar. Der Pfropfen läuft von der Stauchkammer zwischen den seitlichen Führungsmitteln um einen Teil des Umfanges der Pfropfenförderwalze und wird dann auf ein weiteres Förderelement weitergegeben, wo er gekühlt und schliesslich zu einem texturierten Garn aufgelöst wird.

Der Faden wird mittels Fördermedium durch den Förderkanal und durch die Düsenaustrittsöffnung in die Stauchkammer gefördert. Unmittelbar ausserhalb der Düsenaustrittsöffnung entspannt sich das Fördermedium. Der Faden prallt auf den sich in der Stauchkammer bildenden Pfropfen auf und wird dabei gekräuselt. Der Pfropfen in der Stauchkammer wird nach dem Einführen des Fadens bei Produktionsbeginn durch eine temporäre Bremskraft, beispielsweise einen gegen den Faden gerichteten Luftstrahl initiiert. Während dem Betrieb besteht ein Gleichgewicht zwischen der den Pfropfen stossenden Staudruckkraft des Fördermediums und den den Pfropfen bremsenden Reibungskräften an den Wandungen der Stauchkammer, das zu einer kontinuierlichen Pfropfenbildung und einer konstanten Pfropfengeschwindigkeit in der Stauchkammer führt.

Die Qualität des texturierten Garnes hängt eng mit der Gleichmässigkeit der Kräuselung, das heisst mit der Gleichmässigkeit der Pfropfenbildung zusammen. Fehlt der Pfropfen, wird das Garn nicht gekräuselt. Beginnt die Pfropfenbildung zu weit von der Düsenaustrittsöffnung entfernt, wird der Pfropfen weniger dicht, sodass die Kräuselung nicht mehr genügend stark und genügend permanent wird. Dies bedeutet, dass für eine hohe Garnqualität Lage, Konsistenz und Geschwindigkeit des Pfropfens möglichst konstant gehalten werden müssen.

In allen bekannten Vorrichtungen zur Kräuselung von Fäden aus thermoplastischem Material mit Hilfe einer Texturierdüse und einer Stauchkammer können aber Unregelmässigkeiten in der Pfropfenbildung entstehen, vor allem Zustände, in denen die Pfropfenbildung sich zu weit von der Düsenaustrittsöffnung entfernt oder sich gar kein Pfropfen bildet, sogenannte Ausblaser. Je nach Vorrichtung sind diese Ausblaser temporär, das heisst, sie bilden sich ohne Einflussnahme zurück oder sie sind stationär, das heisst, die Maschine muss gestoppt werden, um wieder eine reguläre Pfropfenbildung zu erhalten. Da Fehler in der Pfropfenbildung mit den Verfahren und den Vorrichtungen gemäss dem Stande der Technik nur visuell entdeckt werden können und dadurch oft nicht oder spät entdeckt werden, können Spulen des texturierten Garnes Fehlerstellen aufweisen, die von unentdeckten, temporären Ausblasern herrühren und die erst an einem aus dem Garn hergestellten Produkt erkannt werden. Durch stationäre Ausblaser, die erst nach einer Weile entdeckt werden, können grosse Mengen von Ausschussgarn produziert werden.

Es ist nun die Aufgabe der Erfindung, ein Verfahren und eine Vorrichtung zum kontinuierlichen Kräuseln von Fäden aus thermoplastischem Material aufzuzeigen, mit denen Einbussen in der Garnqualität und Produktion von Ausschuss bedingt durch eine unstabile Pfropfenbildung, insbesondere bedingt durch Ausblaser, vermieden werden können.

Diese Aufgabe wird gelöst durch das Verfahren und die Vorrichtung gemäss den entsprechenden unabhängigen Patentansprüchen.

Die Erfindung beruht auf einer kontinuierlichen und automatischen Kontrolle der Pfropfenbildung die zu Regulierungs- oder Alarmzwecken ausgenützt wird. Diese erfolgt durch sensorische Überwachung des Bereiches der Düsenaustrittsöffnung, beispielsweise durch Messung des statischen Druckes oder einer mit dem statischen Druck korrelierten Grösse im Förderkanal nahe der Düsenaustrittsöffnung oder unmittelbar ausserhalb der Düsenaustrittsöffnung oder durch optische Überwachung der Stauchkammer nahe der Düsenaustrittsöffnung und durch Weiterverarbeitung der durch die sensorische Überwachung erhaltenen Signale zu Regel-, Steuer- und/oder Alarmzwecken.

Der statische Druck im Förderkanal ist die Differenz zwischen dem Gesamtdruck, der im wesentlichen konstant ist, und dem dynamischen Druck, der zum Quadrat der Strömungsgeschwindigkeit proportional ist. Bei leerem Förderkanal (ohne Faden), durch den das Fördermedium durch keinen Faden gehindert strömen kann, ist der statische Druck am geringsten, da die Strömungsgeschwindigkeit hoch ist. Fördert das Medium einen Faden durch den Förderkanal, ist der stationäre Druck verglichen mit dem stationären Druck im fadenfreien Kanal höher, weil das Medium sich am Faden staut. Entsteht vor der Düsenaustrittsöffnung in der Stauchkammer ein Pfropfen, wird das Medium noch stärker gestaut und der statische Druck wird entsprechend höher. Dabei ist der statische Druck umso höher, je näher an der Düsenaustrittsöffnung die Pfropfenbildung beginnt. Die Messung des statischen Druckes im Förderkanal nahe der Düsenaustrittsöffnung kann also direkt als Angabe für den Zustand der Pfropfenbildung ausgewertet werden.

Etwa dasselbe gilt für den statischen Druck in der Stauchkammer, unmittelbar ausserhalb der Düsenaustrittsöffnung.

In derselben Weise kann die Pfropfenbildung durch optische Sensoren in der Stauchkammer überwacht werden. Für eine gute Kräuselung muss die Pfropfenbildung möglichst nahe an der Düsenaustrittsöffnung, höchstens um einen vorgegebenen, empirisch ermittelten Abstand davon entfernt, beginnen. Entfernt sich der Pfropfenanfang weiter von der Düsenaustrittsöffnung, handelt es sich um einen Ausblaser. Mit einem optischen Sensor, der die Stauchkammer im Bereiche des maximal tolerierbaren Abstandes des Pfropfens von der Düsenaustrittsöffnung überwacht, kann ein Ausblaser festgestellt werden.

Da die Pfropfenbildung von der Bremsung in der Stauchkammer und von der Pfropfenweiterförderung abhängig ist, kann sie konstant gehalten werden durch Regulierung dieser Grössen. Das heisst die Überwachung der Pfropfenbildung, insbesondere die Überwachung des Druckes im Bereiche der Düsenaustrittsöffnung kann als Messglied in einen Regelkreis mit einem Proportional/Integral-Regler integriert werden, dessen Stellglieder die Bremswirkung der Stauchkammerwandungen und/oder die Weiterförderung, insbesondere die Weiterförderungsgeschwindigkeit des Pfropfens beeinflussen.

Das erfindungsgemässe Verfahren und beispielhafte Ausführungsformen der erfindungsgemässen Vorrichtung sollen nun anhand der folgenden Figuren detailliert beschrieben werden. Dabei zeigen:
- **Figur 1**: ein Schema der erfindungsgemässen Vorrichtung zur Erläuterung des erfindungsgemässen Verfahrens;
- **Figur 2**: den Verlauf des Messsignales einer Druckmessung im Förderkanal beim Anfahren der Vorrichtung und während ihrem Betrieb;
- **Figur 3**: den Verlauf des Messsignales einer Druckmessung im Förderkanal mit Regelkreis, Warnband und Alarmband;
- **Figur 4**: den Verlauf des Messsignales eines optischen Sensors in der Stauchkammer beim Anfahren der Vorrichtung und während ihrem Betrieb;
- **Figuren 5a und 5b**: eine Texturierdüse mit Stauchkammer mit einer Messöffnung zur Messung des statischen Druckes im Förderkanal und Mitteln zur optischen Überwachung der Stauchkammer;
- **Figur 6**: eine Texturierdüse mit Stauchkammer mit Mitteln zur Überwachung des Druckes in der Stauchkammer.

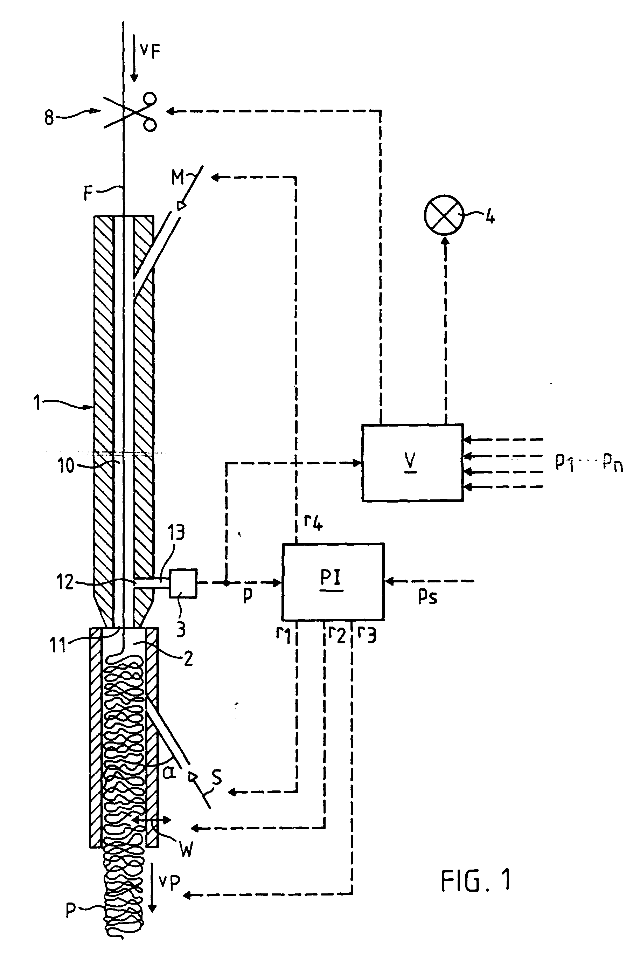

**Figur 1** zeigt ein Schema der erfindungsgemässen Vorrichtung, anhand dessen das erfindungsgemässe Verfahren erläutert werden soll. Die Vorrichtung weist eine Texturierdüse 1 mit einem Förderkanal 10 und einer Düsenaustrittsöffnung 11 auf und eine an diese anschliessende Stauchkammer 2, die entlang dem Verlauf eines zu texturierenden Fadens F geschnitten dargestellt sind. Der Faden F wird mit Hilfe eines Fördermediums M, das unter Druck in den Förderkanal eingespeist wird, mit der Fadengeschwindigkeit V_{F} durch den Förderkanal 10 und aus der Düsenaustrittsöffnung 11 gefördert. Dabei hat das Fördermedium eine erhöhte Temperatur, um den Faden gleichzeitig zu erhitzen. Das Fördermedium M wird unter Druck in den Förderkanal 10 eingespeist und entspannt sich ausserhalb der Düsenaustrittsöffnung 11. Der Faden F wird vom Fördermedium durch den Förderkanal gefördert und prallt ausserhalb der Düsenaustrittsöffnung auf den Pfropfen P, der mit einer Pfropfengeschwindigkeit v_{P} weitergefördert wird, um in weiteren Schritten abgekühlt und dann zu einem texturierten Faden aufgelöst zu werden.

In die Stauchkammer 2 kann mit einem Wikel α zur Pfropfenbewegungsrichtung ein Staumedium S unter Druck gegen den Faden eingespeist werden. Der Winkel α ist dabei zwischen 0 und 90° zu wählen, derart, dass die Strömungsrichtung des Staumediums keine Komponente in Richtung der Pfropfengeschwindigkeit aufweist. Das Staumedium S wird vor allem beim Anfahren der Vorrichtung dazu benützt, durch zusätzliche Bremsung die Pfropfenbildung zu initiieren, indem durch das Staumedium der Faden gebremst und gegen die Wandungen der Stauchkammer bewegt und dadurch zusätzlich durch Reibung an diesen Wandungen gebremst wird.

In der Wandung des Förderkanales ist eine Messöffnung 12 angebracht, an die sich ein Messhohlraum 13 anschliesst. Der Messhohlraum 13 ist bis auf die Messöffnung geschlossen und weist ein Druckmesselement 3, beispielsweise ein Piezoelement auf, mit dem der Druck im Messhohlraum 13 gemessen wird, der dem statischen Druck im Förderkanal (Bereich der Messöffnung) entspricht.

Der Messwert p des Druckmesselementes 3, der dem statischen Druck im Förderkanal entspricht, wird als Messgrösse in einen Proportional/Integral-Regler PI gespeist und/oder in eine Vergleichereinheit V. Mit dem Ausgangssignal (r₁, r₂, r₃ oder r₄) des Reglers PI kann die ärodynamische Bremswirkung in der Stauchkammer durch Regulierung der Zufuhr des Staumediums S (r₁), die mechanische Reibung in der Stauchkammer durch Regulierung W der Stauchkammerwandung oder Stauchkammergeometrie (r₂), die Pfropfengeschwindigkeit durch Regulierung der Geschwindigkeit eines an die Stauchkammer anschliessenden Pfropfenfördermittels (r₃) oder die Stauchwirkung durch Regulierung der Zufuhr des Fördermediums M (r₄) derart beeinflusst werden, dass der Staudruck p einem Soliwert pₛ entspricht. Der Solldruck pₛ kann durch Versuche ermittelt und in den Regler eingegeben werden oder durch eine Eichmessung bestimmt werden.

Die Stellglieder (in der Figur nicht dargestellt) für den Regelkreis sind beispielsweise Regulierventile für die Zufuhr des Staumediums oder des Fördermediums, ein Antrieb, mit dem ein Bremskörper in die Stauchkammer bewegt oder die Stauchkammer irisähnlich verengt wird oder der Antrieb eines gegebenenfalls vorgesehenen an die Stauchkammer anschliessenden Pfropfenfördermittels. Die Regulierung der Zufuhr des Staumediums eignet sich vor allem in Vorrichtungen mit Stauchkammern mit teilweise sich bewegenden Wandungen, die Regulierung der Stauchkammerwandung oder deren Geometrie (beispielsweise der Konizität der Stauchkammer) eignet sich vor allem für Vorrichtungen mit nur stationären Stauchkammerwandungen, wobei die Grössenordnungen der notwendigen Veränderungen sehr klein (im Bereiche von Zehntelsmillimetern) sind. Eine Iris-artige Bewegung ist vor allem für aus einzelnen, stationären Lamellen gebildeten Stauchkammern günstig. Die Regulierung der Pfropfenfördergeschwindigkeit ist nur möglich, wenn die Kräuselvorrichtung ein an die Stauchkammer anschliessendes Pfropfenfördermittel, beispielsweise eine Nadelwalze, aufweist. Die Regulierung der Zufuhr des Fördermediums ist weniger vorteilhaft, da sie sich auch auf die Fadentemperatur und dadurch auf die Kräuselung auswirken und zu Qualitätsschwankungen führen kann.

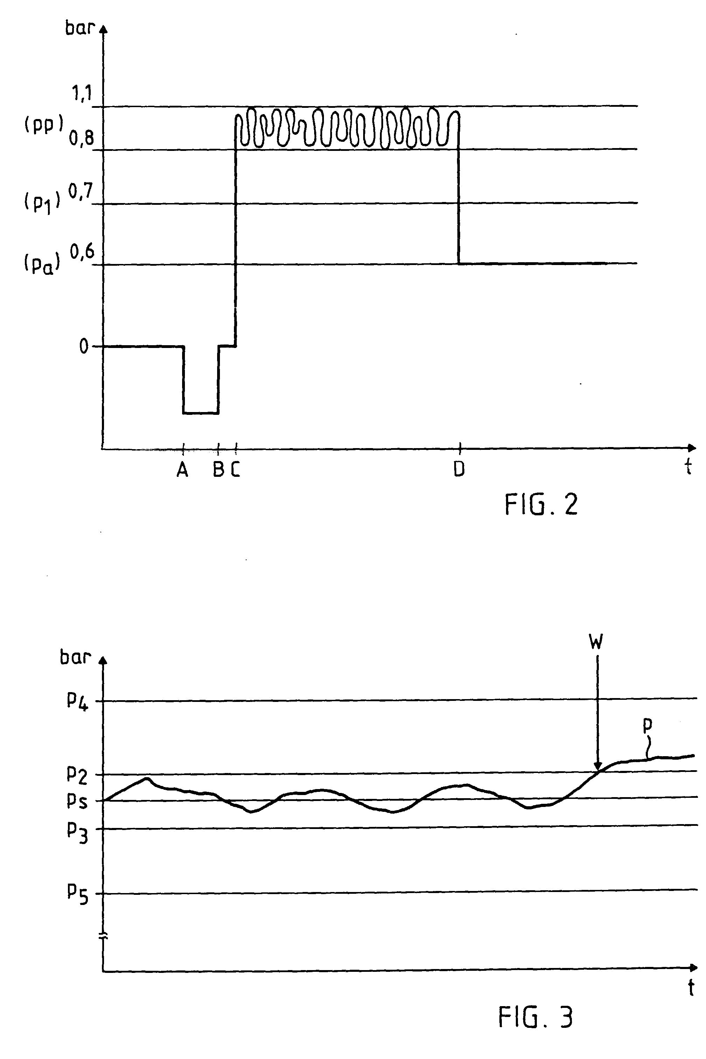

Der durch die Druckmessung erzeugte Messwert kann auch in einer Vergleichereinheit V mit mindestens einem Grenzmesswert (p₁ ...pₙ) verglichen werden. Werden Grenzwerte überschritten, kann beispielsweise eine Alarmleuchte 4 angesteuert werden oder die Produktion durch Fadenunterbruch (8) gestoppt werden. Die Funktion der Vergleichereinheit soll noch im Zusammenhang mit den Figuren 2,3 und 4 detailliert beschrieben werden.

Für die Funktion des Reglers PI kann ein handelsüblicher Proportional/IntegralRegler eingesetzt werden. Für eine kombinierte Regel- und Vergleichs-Funktion kann ein Integralregler beispielsweise mit Alarm- und Stopp-Band eingesetzt werden. Soll nur verglichen und nicht geregelt werden, kann für die Funktion des Vergleichers V beispielsweise eine Schaltung mit einem Diskriminator mit einstellbarem Schwellenwert eingesetzt werden. Die Schwellenwerte werden dabei durch Versuche eingestellt. Selbstverständlich kann die Regel- und/oder Vergleichsfunktion auch softwaremässig realisiert werden.

**Figuren 2,3 und 4** zeigen nun beispielhafte Verläufe eines Messsignals. Auf der Ordinate ist das Messsignal bzw. die dem Messsignal entsprechende physikalische Grösse aufgetragen, auf der Abszisse die Zeit.

**Figur 2** zeigt den Messsignalverlauf für eine Anordnung mit Druckmessung im Förderkanal und Auswertung des Messsignales mit einer Vergleichereinheit. Der statische Druck (in bar Über- bzw. Unterdruck) im Förderkanal, der dem Messwert p (beispielsweise elektrische Spannung) entspricht ist dabei über einer Zeitachse t aufgetragen, wobei die dargestellte Zeit das Anlaufen der Vorrichtung, stationären Betrieb und das Auftreten eines Ausblasers enthält.

Bis zum Zeitpunkt A strömt durch den Förderkanal kein Fördermedium, wobei dieser geschlossen oder für Vorbereitungsarbeiten offen sein kann, das heisst entlang dem Fadenlauf in zwei Kanalteile getrennt ist. Bis zu diesem Zeitpunkt ist der statische Druck in der Düse gleich Atmosphärendruck, der gemessene Druck also gleich Null. Zum Zeitpunkt A wird der Förderkanal geschlossen und das Fördermedium eingeschaltet, sodass es den Kanal durchströmt. Der statische Druck im Kanal sinkt und bleibt während der folgenden Aufheizzeit konstant. Wenn der Kanal Texturiertemperatur erreicht hat (Zeitpunkt B), wird das Fördermedium wieder gestoppt, der Kanal geöffnet und der Faden eingezogen. Zum Zeitpunkt C wird der Kanal geschlossen und sofort die Pfropfenbildung initiiert, beispielsweise durch kurzzeitige Fadenbremsung mit Hilfe des Staumediums S. Durch die Stauung des Fördermediums am Faden im Förderkanal und am Pfropfen in der Stauchkammer steigt der statische Druck im Förderkanal und pendelt sich bei kontinuierlichem und regelmässigem Betrieb in einem Druckbereich entsprechend einem Messwertbereich pp ein. Der Betrieb kann als optimal bezeichnet werden, wenn der Messwertbereich pp möglichst schmal ist und über lange Zeiträume konstant bleibt.

Im Zeitpunkt D tritt nun ein Ausblaser auf, das heisst die Stelle der Pfropenbildung entfernt sich von der Düsenaustrittsöffnung. Dadurch wird die Stauwirkung des Pfropfens kleiner und der gemessene statische Druck fällt, und zwar auf einen Druck entsprechend einem Messwert pₐ, der im Extremfall dem statischen Druck im Förderkanal ganz ohne Stauwirkung eines Pfropfens entspricht.

Mit einer Messung in der Art nach Figur 2, wobei der Messwert p verfolgt und die Pfropfenbildung beobachtet wird, kann ein Schwellenmesswert p₁ bestimmt werden, derart, dass Unregelmässigkeiten in der Pfropfenbildung soweit tolerierbar sind, solange sie keine Messwertschwankungen bedingen, die zu einer Unterschreitung des Schwellenmesswertes p₁ führen. Der Schwellenmesswert p₁ wird höher angesetzt als der Messwert pₐ für einen Ausblaser. Der Schwellenmesswert p₁ wird genügend tief angesetzt, dass er einen genügenden Abstand vom Messwertbereich pp hat, derart, dass bei regelmässigem Betrieb die Messwerte p nicht in seinen Bereich fallen. Der Schwellenmesswert p₁ wird genügend hoch angesetzt, dass Pfropfenunregelmässigkeiten, die zu Qualitätseinbussen und/oder zu permanenten Ausblasern führen, als solche erkannt werden.

Für eine Vorrichtung entsprechend der eingangs bereits erwähnten Kräuselvorrichtung analog der europäischen Patentschrift Nr. 310890 ergaben sich beispielsweise folgende Druckverhältnisse: Bei einem Speisedruck des Fördermediums von 7 bis 7,5 bar lag der Druckbereich bei regelmässiger Pfropfenbildung (Messwertbereich pp) zwischen 0,8 bis 1,1 bar (Überdruck), der gemessene Druck bei einem Ausblaser (Messwert pₐ) betrug 0,6 bar, sodass der Schwellendruck (Schwellenmesswert p₁) bei ca. 0,7 bar angesetzt werden musste.

**Figur 3** zeigt einen beispielhaften Signalverlauf für eine Anordnung mit Messung des Druckes im Förderkanal, mit Regelkreis, Warnband (p₂/p₃) und Alarmband (p₄/p₅) Bei optimalem Betrieb sollte der geregelte Messwert innerhalb des Warnbandes liegen. Liegt der Messwert ausserhalb des Warnbandes, aber noch innerhalb des Alarmbandes kann noch ohne Qualitätseinbusse produziert werden aber es wird eine Warnung W erzeugt (Warnleuchte, Protokollausdruck), die auf eine bald notwendige Revision (Reinigung) hinweist. Steigt der gemessene Druck über p₄ ist die Düsenaustrittsöffnung verstopft, sinkt er unter p₅, liegt ein Ausblaser vor. In beiden Fällen muss die Produktion beispielsweise durch Fadenschnitt gestoppt werden.

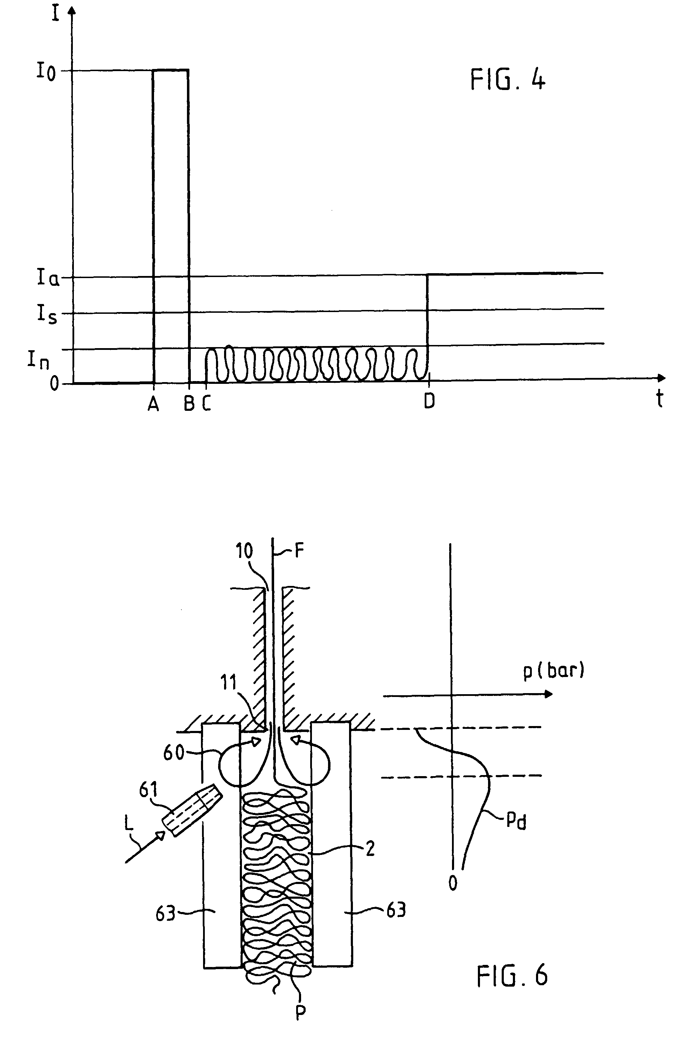

**Figur 4** zeigt den Verlauf des Messsignales I eines optischen Sensors in der Stauchkammer über den gleichen Zeitverlauf aufgetragen wie Figur 1. Das Messsignal I ist beispielsweise das Messsignal eines optischen Sensors bestehend aus Lichtquelle und lichtempfindlicher Zelle, die einander gegenüberliegend in der Stauchkammer angebracht sind. Das von der Lichtquelle emittierte Licht ist gegen die lichtempfindliche Zelle gerichtet, wird aber von Faden und/oder Pfropfen teilweise absorbiert und gestreut. Das Messsignal entspricht der Intensität des von der lichtempfindlichen Zelle empfangenen Lichtes. Diese ist hoch bei fadenloser Stauchkammer (I₀), niedriger bei im wesentlichen gradlinig durchlaufendem Faden (Iₐ) bedingt durch Absorption durch den Faden, was einem Ausblaser entspricht, und sehr niedrig bei einem Pfropfen in der Stauchkammer (Bereich Iₙ) Der Schwellenmesswert Iₛ wird angesetzt zwischen Iₐ und der oberen Grenze von Iₙ.

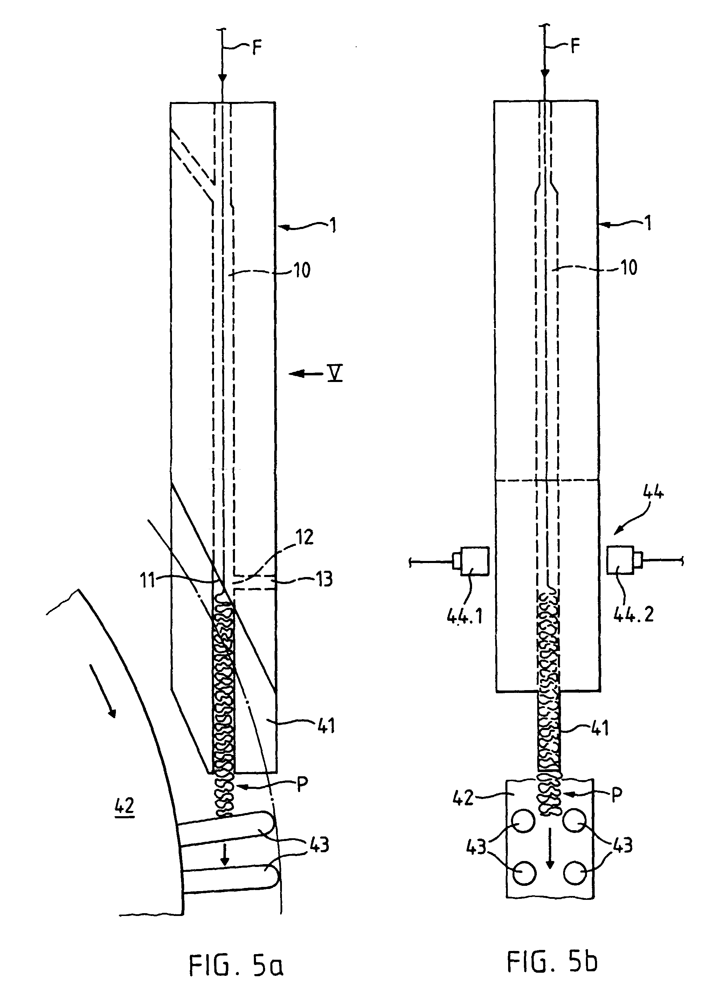

**Figuren 5a und 5b** zeigen als zwei beispielhafte Ausführungsformen der erfindungsgemässen Kräuselvorrichtung je eine Texturierdüse 1 mit Anformungen 41, die die Funktion der Stauchkammer übernehmen. Die Texturierdüse ist in der Figur 5b gegenüber der Figur 5a um 90° gedreht (Blickrichtung wie Pfeil V in Figur 5a). Der Faden F wird wie beschrieben durch den Förderkanal 10 und durch die Düsenaustrittsöffnung 11 gefördert. Unmittelbar ausserhalb der Düsenaustrittsöffnung beginnt die Pfropfenbildung. Der gebildete Pfropfen P wird mit Hilfe einer Propfenförderwalze 42 zwischen Zähnen 43 von der Texturierdüse weg gefördert.

In der Vorrichtung gemäss Figur 5a wird der Staudruck im Förderkanal gemessen. Die Vorrichtung zeigt eine Messöffnung 12, die in einen Messhohlraum 13 führt. Der Messhohlraum kann ausserhalb der Wandung des Förderkanales beliebige, der Gesamtanordnung angepasste Formen annehmen. Das Druckmesselement (in der Figur nicht dargestellt) ist vorteilhafterweise ausserhalb der Förderkanalwandungen angebracht.

In der Vorrichtung gemäss Figur 5b wird die Pfropfenbildung optisch überwacht. Die Vorrichtung zeigt eine Lichtschrankenanordnung 44, die alternativ zu Messhohlraum und Druckmesselement vorgesehen sein kann. Es handelt sich beispielsweise um eine Lichtquelle 44.1 und einen Empfänger 44.2, die einander gegenüberliegend auf den offenen Seiten der Stauchkammer derart angebracht sind, dass der Empfänger das Licht der Lichtquelle empfängt.

**Figur 6** zeigt schematisch eine weitere beispielhafte Anordnung zur Überwachung des Druckes im Bereiche der Düsenaustrittsöffnung. Es handelt sich um die Messung des dynamischen Druckes in einer Zuleitung für Messluft in die Stauchkammer und zwar unmittelbar ausserhalb der Düsenaustrittsöffnung.

Die Figur zeigt wiederum einen Förderkanal 10, in dem ein Faden F gefördert wird und eine Stauchkammer 2, in der sich ein Pfropfen P bildet. Die Stauchkammer 2 ist in der dargestellten beispielhaften Ausführungsform begrenzt durch radial zum Pfropfen angeordnete Lamellen 63. Im Bereiche der Düsenaustrittsöffnung 11 entspannt sich das Fördermedium zwischen die Lamellen. Messungen zeigen, dass sich zwischen Pfropfen P und Düsenaustrittsöffnung 11 Wirbel (Pfeile 60) bilden, derart, dass nahe an der Düsenaustrittsöffnung eine Strömung gegen den Faden, nahe am Pfropfen eine Strömung aus der Stauchkammer entsteht. Die Form dieser Wirbel ist stark von der geometrischen Ausgestaltung von Düsenaustrittsöffnung und Stauchkammer abhängig. Wird nun im Bereiche zwischen Düsenaustrittsöffnung und Pfropfenanfang mittels einer fluidischen Düse der Druck an der Staukammerwand in Abhängigkeit der Distanz von der Düsenaustrittsöffnung gemessen, so ergibt sich, wie neben dem Vorrichtungsschema der Figur 6 dargestellt, unmittelbar ausserhalb der Düsenaustrittsöffnung ein Unterdruck (Sog), der über eine neutrale Zone zu einem Druckmaximum beim Pfropfenanfang ansteigt. Wird nun beispielsweise in dem Abstand von der Düsenaustrittsöffnung, in dem bei optimaler Produktion der Pfropfen beginnt, eine derartige fluidische Düse 61 installiert, kann aus dem in der Düse gemessenen Druck eine Aussage gemacht werden über die Position des Pfropfens. Ein derartiges Messsignal kann in analoger Weise zum Messsignal des Sensors für den Staudruck im Förderkanal ausgewertet werden.

Unter fluidischer Düse wird eine Messdüse verstanden, durch die mit konstanter Leistung Messluft strömt und in der der Staudruck gemessen wird. Eine derartige fluidische Düse erweist sich im Bereiche der Stauchkammer als besonders vorteilhaft, da sie dank dem Messluftstrom in hohem Grade selbstreinigend ist.

Vorteilhafterweise ist der Messhohlraum bzw. die Mittel zur Überwachung der Stauchkammer, beispielsweise die Lichtschranke, so nahe wie möglich an der Düsenaustrittsöffnung vorgesehen.

## Patentansprüche

1. Verfahren zum kontinuierlichen Kräuseln eines Fadens (F) aus einem thermoplastischen Material, wobei der Faden erwärmt, mit Hilfe eines strömenden Fördermediums (M) mit einer Fadengeschwindigkeit (v_{F}) durch einen Förderkanal (10) und durch eine Düsenaustrittsöffnung (11) in eine Stauchkammer (2) gefördert, durch Bremsung in der Stauchkammer zu einem Pfropfen (P) gestaucht und in dieser Form mit einer Pfropfengeschwindigkeit (v_{P}), die kleiner ist als die Fadengeschwindigkeit (v_{F}), weitergefördert wird zu Kühlung und Auflösung, **dadurch gekennzeichnet**, dass im Bereiche der Düsenaustrittsöffnung (11) die Pfropfenbildung sensorisch überwacht wird, und dass die durch die Überwachung erzeugten Messsignale als Messgrössen für einen geschlossenen Regelkreis zur Konstanthaltung der Pfropfenbildung oder zur Ansteuerung von Stopp-, Alarm- oder Warnvorrichtungen oder gleichzeitig für die Regulierung und die Ansteuerung weiterverarbeitet werden.

2. Verfahren nach Anspruch 1, **dadurch gekennzeichnet**, dass die Pfropfenbildung überwacht wird durch Messung des statischen Druckes im Förderkanal (10) im Bereiche der Düsenaustrittsöffnung (11).

3. Verfahren nach Anspruch 1, **dadurch gekennzeichnet**, dass die Pfropfenbildung überwacht wird durch die Messung des Druckes in einer fluidischen Düse (61), die im Bereiche der Düsenaustrittsöffnung in die Stauchkammer mündet.

4. Verfahren nach Anspruch 1, **dadurch gekennzeichnet**, dass die Pfropfenbildung überwacht wird durch optische Überwachung des Stauchkammerinnenraumes im Bereiche der Düsenaustrittsöffnung (11).

5. Verfahren nach einem der Ansprüche 1 bis 3, **dadurch gekennzeichnet**, dass die Geschwindigkeit, mit der der Pfropfen durch ein Pfropfenfördermittel aus der Stauchkammer ausgetragen wird, Stellgrösse des Regelkreises ist.

6. Verfahren nach einem der Ansprüche 1 bis 3, **dadurch gekennzeichnet**, dass in die Stauchkammer ein Staumedium zugeführt wird und dass die zugeführte Menge an Staumedium die Stellgrösse des Regelkreises ist.

7. Verfahren nach einem der Ansprüche 1 bis 3**, dadurch gekennzeichnet**, dass die Stauchkammer einen relativ zum Pfropfen verstellbaren Wandteil aufweist und dass die Position dieses Wandteiles die Stellgrösse des Regelkreises ist.

8. Verfahren nach einem der Ansprüche 1 bis 3, **dadurch gekennzeichnet**, dass die Stauchkammerwandung aus einzelnen Lamellen besteht, die irisartig gegeneinander beweglich sind, und dass der Querschnitt und die Konizität der Stauchkammer Stellgrösse des Regelkreises ist.

9. Verfahren nach einem der Ansprüche 1 bis 3**, dadurch gekennzeichnet**, dass die Zufuhrmenge an Fördermedium die Stellgrösse des Regelkreises ist.

10. Verfahren nach einem der Ansprüche 1 bis 9, **dadurch gekennzeichnet**, dass die von der Überwachung der Pfropfenbildung erzeugten Messwerte durch Vergleichen mit Schwellenwerten zu Steuersignalen zum Ansteuern von Alarmelementen weiterverarbeitet werden.

11. Verfahren nach einem der Ansprüche 1 bis 10, **dadurch gekennzeichnet**, dass die von der Überwachung der Pfropfenbildung erzeugten 1 Messwerte durch Vergleichen mit Schwellenwerten zur Ansteuerung einer Vorrichtung, die den Faden schneidet oder die Maschine stoppt, weiterverarbeitet werden.

12. Verfahren nach einem der Ansprüche 9 oder 11, **dadurch gekennzeichnet**, dass der Vergleich zwischen dem Messwert der sensorischen Überwachung und dem Schwellenmesswert mit einem Diskriminator hardwaremässig oder nach einer analog/digital-Wandlung softwaremässig durchgeführt wird.

13. Vorrichtung zur Durchführung des Verfahrens nach einem der Ansprüche 1 bis 12, die eine Texturierdüse (1) mit einem Förderkanal (10) aufweist mit einer Einlassöffnung für einen Faden, mit einem Einlass für ein Fördermedium (M) und mit einer Düsenaustrittsöffnung (11) für Faden und Fördermedium und eine in der die Stauchung des Fadens zu einem Pfrofen erfolgt Stauchkammer (2), **dadurch gekennzeichnet**, dass im Bereich der Düsenaustrittsöffnung (11) sensorische Mittel zur Überwachung der Pfropfenbildung vorhanden sind.

14. Vorrichtung nach Anspruch 13, **dadurch gekennzeichnet**, dass die sensorischen Mittel ein Messhohlraum (13) mit einem Druckmesselement (3) sind, wobei der Messhohlraum (13) im Bereiche der Düsenaustrittsöffnung (11) mit einer Messöffnung (12) in den Förderkanal (1) mündet.

15. Vorrichtung nach Anspruch 13, **dadurch gekennzeichnet**, dass die sensorischen Mittel ein optischer Sensor sind, der den Innenraum der Stauchkammer überwacht.

16. Vorrichtung nach Anspruch 13, **dadurch gekennzeichnet**, dass die sensorischen Mittel eine fluidische Düse (61), die in die Stauchkammer (2) mündet aufweist und in deren Bereich ein Druckmesselement angeordnet ist.

17. Vorrichtung nach einem der Ansprüche 13 bis 16, **dadurch gekennzeichnet**, dass sie einen Proportional/Integral-Regler aufweist.

## Claims

1. A method for continual crimping of a yarn (F) made from a thermoplastic material, with the yarn being heated, conveyed through a conveying duct (10) and through a nozzle exit aperature (11) into a stuffer box (2) with the help of a flowing conveying medium (M) with a yarn speed (V_{F}), crimped into a plug (P) in the stuffer box by decelaration and being further conveyed towards cooling and opening in this form with a plug speed (Vp) which is smaller than the yarn speed (V_{F}), characterized in that in the zone of the nozzle exit aperture (11) the formation of the plug is monitored by sensors and that the measured signals generated by the monitoring are further processed as measured values for a closed control loop for keeping constant the plug formation or for controlling stop, alarm or warning apparatuses or simultaneously for the regulation and the control.

2. A method as claimed in claim 1, characterized in that the plug formation is monitored by the measurement of the static pressure in the conveying duct (10) in the zone of the nozzle exit aperture (11).

3. A method as claimed in claim 1, characterized in that the plug formation is monitored by the measurement of the pressure in a fluidic nozzle (61) which opens out into the stuffer box in the zone of the nozzle exit aperture.

4. A method as claimed in claim 1, characterized in that the plug formation is monitored by optical monitoring of the inner space of the stuffer box in the zone of the nozzle exit aperture (11).

5. A method as claimed in one of the claims 1 to 3, characterized in that the speed with which the plug is carried off from the stuffer box by a plug conveying means is a manipulated variable of the control loop.

6. A method as claimed in one of the claims 1 to 3, characterized in that a stuffer medium is supplied into the stuffer box and that the supplied stuffer medium quantity is the manipulated variable of the control loop.

7. A method as claimed in one of the claims 1 to 3, characterized in that the stuffer box comprises a wall element which is adjustable relative to the plug and that the position of said wall element is the manipulated variable of the control loop.

8. A method as claimed in one of the claims 1 to 3, characterized in that the side walls of the stuffer box consist of individual lamellae which are mutually movable in an iris-like manner and that the cross section and conicity of the stuffer box is the manipulated variable of the control loop.

9. A method as claimed in one of the claims 1 to 3, characterized in that the supplied quantity of conveying medium is the manipulated variable of the control loop.

10. A method as claimed in one of the claims 1 to 9, characterized in that the measured values generated by the monitoring of the plug formation are further processed into control signals for controlling alarm elements by comparing them with critical values.

11. A method as claimed in one of the claims 1 to 10, characterized in that the measured values generated by the monitoring of the plug formation are further processed by comparison with critical values for controlling an apparatus which cuts the yarn or stops the machine.

12. A method as claimed in one of the claims 9 or 11, characterized in that the comparison between the measured value of the monitoring by sensors and the critical measured value is carried out with a discriminator by way of hardware or, after an analog-to-digital conversion, by way of software .

13. An apparatus for carrying out the method as claimed in one of the claims 1 to 12, comprising a texturing nozzle (1) with a conveying duct (1) with an inlet opening for a yarn, with an inlet for a conveying medium (M) and with a nozzle exit aperture (11) for the yarn and conveying medium and a stuffer box (2) in which the crimping of the yarn into a plug occurs, characterized in that sensor means for monitoring the plug formation are present in the zone of the nozzle exit aperture (11).

14. An apparatus as claimed in claim 13, characterized in that the sensor means are a hollow measuring chamber (13) with a pressure gauge element (3), with the hollow measuring chamber (13) opening into the conveying duct (1) with a measuring aperture (12) in the zone of the nozzle exit aperture (11).

15. An apparatus as claimed in claim 13, characterized in that the sensor means are an optical sensor which monitors the inner space of the stuffer box.

16. An apparatus as claimed in claim 13, characterized in that the sensor means is provided with a fluidic nozzle (61) which opens out into the stuffer box (2) and in the zone of which there is arranged a pressure gauge element.

17. An apparatus as claimed in one of the claims 13 to 16, characterized in that it comprises a proportional/integral controller.

## Revendications

1. Procédé servant à friser continuellement un fil (F) constitué par une matière thermoplastique, et où le fil est échauffé, est transporté avec une vitesse de fil (v_{F}), à l'aide d'un média de transport en mouvement (M), à travers un canal de transport (10) et à travers une ouverture de sortie de buse (11) dans une chambre de compression (2), est comprimé par freinage en un bouchon (P) dans la chambre de compression, et est transporté plus loins sous cette forme avec une vitesse de bouchon (vₚ) qui est plus petite que la vitesse de fil (v_{F}), pour être refroidi et délié,
caractérisé par le fait que
la formation du bouchon est surveillée d'une manière sensorielle dans la zone de l'ouverture de sortie de buse (11), et que les signaux de mesure émis par la surveillance sont traités ultérieurement comme variables mesurées pour une boucle d'asservissement fermée, afin de maintenir constante la formmation du bouchon. ou pour l'excitation des dispositifs d'arrêt, d'alarme ou d'avertissement, ou simultanément pour la régulation et l'excitation.

2. Procédé selon revendication 1,
caractérisé par le fait que
la formation du bouchon est surveillée par mesure de la pression statique régnant dans le canal de transport (10), dans la zone de l'ouverture de sortie de buse (11).

3. Procédé selon revendication 1,
caractérisé par le fait que
la formation du bouchon est surveillée par mesure de la pression régnant dans une buse fluidique (61) qui débouche dans la chambre de compression, dans la zone de l'ouverture de sortie de buse.

4. Procédé selon revendication 1,
caractérisé par le fait que
la formation du bouchon est surveillée par une surveillance optique de l'espace intérieur de la chambre de compression, dans la zone de l'ouverture de sortie de buse (11).

5. Procédé selon une des revendications 1 à 3,
caractérisé par le fait que
la vitesse, avec laquelle le bouchon est évacué hors de la chambre de compression par un moyen de transport de bouchon, sert de variable réglante dans la boucle d'asservissement.

6. Procédé selon une des revendications 1 à 3,
caractérisé par le fait
qu'un média d'accumulation est introduit dans la chambre de compression, et que la quantité de média d'accumulation introduite sert de variable réglante dans la boucle d'asservissement.

7. Procédé selon une des revendications 1 à 3,
caractérisé par le fait que
la chambre de compression possède une partie de paroi qui est ajustable par rapport au bouchon, et que la position de cette partie de paroi sert de variable réglante dans la boucle d'asservissement.

8. Procédé selon une des revendications 1 à 3,
caractérisé par le fait que
la paroi de chambre de compression est constituée par des lamelles individuelles qui sont mobiles l'une contre l'autre, en forme d'iris, et que la section et la conicité de la chambre de compression servent de variable réglante dans la boucle d'asservissement.

9. Procédé selon une des revendications 1 à 3,
caractérisé par le fait que
la quantité d'amenée en média de transport sert de variable réglante dans la boucle d'asservissement.

10. Procédé selon une des revendications 1 à 9,
caractérisé par le fait que
les valeurs mesurées émises par la surveillance de la formation du bouchon sont traitées ultérieurement par comparaison avec des valeurs seuil pour des signaux d'asservissement servant à exciter des éléments d'alarme.

11. Procédé selon une des revendications 1 à 10,
caractérisé par le fait que
les valeurs mesurées émises par la surveillance de la formation du bouchon sont traitées ultérieurement par comparaison avec des valeurs seuil, afin d'exciter un dispositif Qui coupe le fil ou arrête la machine.

12. Procédé selon une des revendications 9 ou 11,
caractérisé par le fait que
la comparaison entre la valeur mesurée de la surveillance sensorielle et la valeur de seuil mesurée est réalisée par un discriminateur type hardware ou selon une transformation analogique/digitale type software.

13. Dispositif servant à la réalisation du procédé selon une des revendications 1 à 12, qui possède une buse de texturation (1) avec un canal de transport (10) ayant une ouverture d'entrée pour un fil, une entrée pour un média de transport (M) et une ouverture de sortie de buse (11) pour le fil et le média de transport, et une chambre de compression (2) dans laquelle a lieu la compression du fil en un bouchon,
caractérisé par le fait que
des moyens sensoriels sont prévus dans la zone de l'ouverture de sortie de buse (11) pour la surveillance de la formation du bouchon.

14. Dispositif selon revendication 13,
caractérisé par le fait que
les moyens sensoriels sont un espace de mesure creux (13) avec un élément de mesure de pression (3), et où l'espace de mesure creux (13) débouche dans le canal de transport (1) avec une ouverture de mesure (12), dans la zone de l'ouverture de sortie de buse (11).

15. Dispositif selon revendication 13,
caractérisé par le fait que
ies moyens sensoriels sont un détecteur optique qui surveille l'espace intérieur de la chambre de compression.

16. Dispositif selon revendication 13,
caractérisé par le fait que
les moyens sensoriels possèdent une buse fluidique (61) qui débouche dans la chambre de compression (2), et un élément de mesure de pression est disposé dans la zone de cette buse.

17. Dispositif selon une des revendications 13 à 16,
caractérisé par le fait
qu'il possède un régulateur proportionnel/intégral.
